# EUROPEAN PATENT SPECIFICATION

(11) **EP 1 544 198 B1**
(45) Date of publication and mention of the grant of the patent: **04.06.2008**
(21) Application number: 04027598.4
(22) Date of filing: 19.11.2004
(51) Int. Cl.: C07D 403/10, A61K 31/4178

(54) **A process for the preparation of crystalline losartan potassium**
Herstellungsverfahren von kristallinem Losartan-Kaliumsalz
Procédé de synthèse de losartan potassique cristallin

(30) Priority: 16.12.2003 IT MI20032472
(43) Date of publication of application: 22.06.2005
(73) Proprietor: Dipharma S.p.A., 33036 Mereto di Tomba (Udine) (IT)
(72) Inventor: Razetti, Gabriele, 20099 Sesto S.Giovanni (MI) (IT); Magrone, Domenico, 20128 Milano (IT); Ercoli, Mauro, 20146 Milano (IT); Allegrini, Pietro, 20097 San Donato Milanese (MI) (IT); Castaldi, Graziano, 28072 Briona (NO) (IT)
(74) Representative: Minoja, Fabrizio

(56) References cited:
- WO-A-95/17396
- WO-A-02/094816
- WO-A-03/048135
- WO-A-20/04066997
- RAGHAVAN K ET AL: "SPECTROSCOPIC INVESTIGATION OF LOSARTAN POLYMORPHS" PHARMACEUTICAL RESEARCH, NEW YORK, NY, US, vol. 10, no. 6, 1993, pages 900-904, XP000915957 ISSN: 0724-8741

## Description

### FIELD OF THE INVENTION

The present invention relates to a process for the preparation of crystalline losartan potassium and crystalline hydrate losartan potassium, comprising the crystallization of losartan potassium from an aprotic solvent.

### TECHNOLOGICAL BACKGROUND

Losartan potassium, namely {2-butyl-5-chloro-3-[2'-(2H-tetrazol-5-yl)-biphenyl-4-ylmethyl]-3H-imidazol-4-yl}-methanol potassium salt, of formula (I) is a known angiotensin II antagonist.

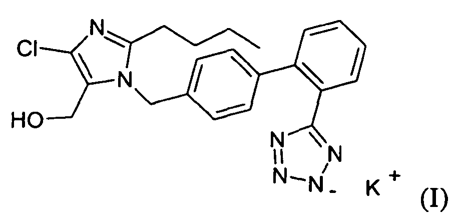

Angiotensin II antagonists are known medicaments used in the treatment of hypertension, anxiety, glaucoma and cardiac attacks. The synthesis of losartan in the free acid form (formula II) or as tetrazolyl-protected derivative is disclosed in US 5,138,069 and WO 93/10106. The advantages provided by pharmaceutical products in the crystalline form in terms of easiness of processes for the preparation of related medicaments are well known. Crystalline compounds are in fact known to be more suited to the formulation of galenic forms, thanks both to their flowability in the form of powders or granulates, and to the surface properties of the crystals which promote adhesion, for example during the preparation of tablets. Furthermore, the solubility of crystalline compounds in aqueous solutions, in particular in the gastric juices, can also be significantly different than that of the corresponding amorphous compounds. There is therefore the need to discriminate between the crystalline and the amorphous forms of biologically active compounds, so as to fulfil the various pharmaceutical requirements.

A number of crystalline and amorphous forms of losartan potassium are known from WO 95/17396 and WO 03/048135. According to WO 95/17396, crystalline losartan potassium is prepared by salification of acid losartan with an alkali hydroxide. The losartan potassium aqueous solution is then added to a isopropanol-cyclohexane azeotropic mixture under reflux. Water is then removed by azeotropic distillation of the resulting water-isopropanol-cyclohexane ternary mixture, which boils at 64°C. When the solution is anhydrous, the head temperature raises to 69°C and losartan potassium crystallizes.

US 5,859,258 discloses another crystallization process which comprises dissolution of losartan potassium in isopropanol-water, distillation of the binary azeotrope to an approx. 2.6% water content, precipitation by addition of a losartan potassium suspension in cyclohexane, subsequent distillation of the ternary azeotrope to a water content ranging from 0.02 to 0.11 %, and finally drying crystalline losartan potassium under vacuum at a temperature of approx. 45-50°C.

The known methods for the preparation of crystalline losartan potassium involve operations which require remarkable precision in the solvents ratios (which ratios are difficult to control on an industrial scale), as well as complex, cumbersome procedures.

There is therefore the need for a process for the preparation of crystalline losartan potassium, which is simpler, easier and fulfils the requirements for the production on an industrial scale.

### SUMMARY OF THE INVENTION

An efficient process has been found for a simple, efficient preparation of losartan potassium in the crystalline form I, known from WO 95/17396 (in the following: crystalline form), and in the crystalline hydrate form having PXRD pattern with the characteristic peaks at about 5.7, 8.9, 13.3, 17.5, 20.0 and 21.1 ± 0.2 degrees 2θ as reported in claim 31 of WO 03/048135 (in the following: crystalline hydrate form).

### DETAILED DISCLOSURE OF THE INVENTION

An object of the invention is a process for the preparation of losartan potassium in the crystalline form or in the crystalline hydrate form, as defined above, comprising the reaction of a dispersion of acid losartan of formula (II), in an organic aprotic solvent, with a potassium basic salt, in the presence of water.

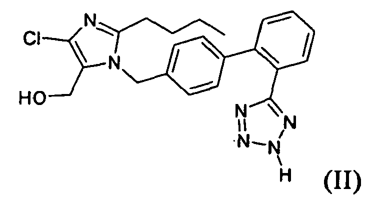

An organic aprotic solvent is preferably a solvent selected from the group comprising acetone, toluene, acetonitrile and ethyl acetate, more preferably ethyl acetate or toluene, in particular ethyl acetate.

A potassium basic salt is for example potassium hydroxide, potassium carbonate or potassium bicarbonate, preferably potassium bicarbonate.

The molar ratio of potassium salt to compound of formula (II) ranges from about 0.8 to 1.5, and is preferably about 1.0.

The weight ratio of water to potassium basic salt ranges from about 0.1 to 5.0, preferably from about 1.0 to 3.0.

The weight ratio of organic aprotic solvent to compound of formula (II) ranges from about 4:1 to 15:1, preferably from about 9:1 to 11:1.

According to a preferred embodiment of the invention, losartan potassium in the crystalline hydrate form is obtained by a process comprising:
a) salification reaction to obtain losartan potassium;
b) cooling of the losartan potassium solution to precipitate losartan potassium crystalline hydrate;
c) filtration of the mixture to isolate crystalline hydrate losartan potassium;
d) washing of the losartan potassium crystalline hydrate with a solvent in which losartan potassium crystalline hydrate is insoluble; and
e) drying of the losartan potassium crystalline hydrate.

According to a further preferred embodiment of the invention, losartan potassium in the crystalline form is obtained by a process comprising:
a) salification reaction to obtain losartan potassium;
a') azeotropic distillation to remove water;
b) cooling of the losartan potassium solution to precipitate crystalline losartan potassium;
c) filtration of the mixture to isolate crystalline losartan potassium;
d) washing of the crystalline losartan potassium with a solvent in which crystalline losartan potassium is insoluble; and
e) drying of the crystalline losartan potassium.

Each of the single steps a), b), c), d) and e), respectively, in both preferred embodiments of the invention above, can be similarly performed.

According to step a), the reaction is carried out at a temperature ranging from about 15°C to the reflux temperature of the reaction mixture, preferably approx. from 40 to 80°C.

After the azeotropic distillation of step a'), the water content of the product is equal to or lower than 1%.

In step b), the losartan potassium solution is cooled to a temperature lower than 0°C, preferably from -2°C to -5°C.

In step d), the solvent can be for example a solvent selected from the group comprising toluene, acetone, acetonitrile and ethyl acetate, in particular the same organic aprotic solvent previously used in step a) above, more preferably ethyl acetate.

In step e), drying is carried out preferably under vacuum at a temperature ranging from about 40 to 55°C.

The following examples further illustrate the invention.

### Example 1. Preparation of losartan potassium, crystalline hydrate form

A suspension of {2-butyl-5-chloro-3-[2'-(2H-tetrazol-5-yl)-biphenyl-4-ylmethyl]-3H-imidazol-4-yl}-methanol (II) (4.2 g) in ethyl acetate (60 ml) is added with a solution of potassium bicarbonate (1g) in water (2.8 g), at 50°C. The resulting clear solution is cooled to -2/-5°C and the precipitate which forms is filtered, washed with 10 ml of ethyl acetate and dried under vacuum at 60°C, thereby obtaining 4.3 g of losartan potassium crystalline hydrate.

NMR: (¹H, DMSO, 300 mHz): δ 0.80 (3H, t, J=10. CH₃), 1.25 (2H, sext, J=10. CH₃CH₂), 1.45 (2H, quin, J=10. CH₃CH₂CH₂), 2.45-2.55 (2H, m, CH₃CH₂CH₂CH₂), 4.25 (2H, d, J= 3, CH₂OH), 5.15-5.25 (3H, m, CH₂Ar and OH), 6.88 (d, 2H, J=12, ArH), 7.08 (d, 2H, J=12, ArH), 7.23-7.36 (3H, m, ArH), 7.50-7.55 (1H, ArH).

### Example 2. Preparation of losartan potassium, crystalline hydrate form

A suspension of {2-butyl-5-chloro-3-[2'-(2H-tetrazol-5-yl)-biphenyl-4-ylmethyl]-3H-imidazol-4-yl}-methanol (II) (4.2 g) in ethyl acetate (60 ml) is added with a solution of potassium hydroxide (0.5 g) in water (0.5 g), at 50°C. The resulting clear solution is cooled to -2/-5°C and the precipitate which forms is filtered, washed with 10 ml of ethyl acetate and dried under vacuum at 60°C, thereby obtaining 4.2 g of losartan potassium crystalline hydrate.

### Example 3. Preparation of losartan potassium, crystalline form

A suspension of {2-butyl-5-chloro-3-[2'-(2H-tetrazol-5-yl)-biphenyl-4-ylmethyl]-3H-imidazol-4-yl}-methanol (II) (4.2 g) in ethyl acetate (60 ml) is added with a solution of potassium bicarbonate (1 g) in water (2.8 g), at 50°C. When the mixture turns to a clear solution, 30 ml of solvent are distilled off, then the solution is cooled to -2/-5°C. The resulting precipitate is filtered, washed with 10 ml of ethyl acetate and dried under vacuum at 60°C, thereby obtaining 4.3 g of losartan potassium crystalline form.

### Example 4. Preparation of losartan potassium, crystalline form

A suspension of {2-butyl-5-chloro-3-[2'-(2H-tetrazol-5-yl)-biphenyl-4-ylmethyl]-3H-imidazol-4-yl}-methanol (II) (4.2 g) in toluene (60 ml) is added with a solution of potassium bicarbonate (1 g) in water (2.8 g), at 50°C. 30 ml of solution are distilled off. The mixture is cooled to -2/-5°C and the resulting precipitate is filtered, washed with 10 ml of toluene and dried under vacuum at 60°C, thereby obtaining 4.4 g of losartan potassium, crystalline form.

## Claims

1. A process for the preparation of losartan potassium in the crystalline form I or in the crystalline hydrate form having PXRD pattern with peaks at about 5.7, 8.9, 13.3, 17.5, 20.0 and 21.1 ± 0.2 degrees 2θ, comprising the reaction of a dispersion of acid losartan, in an organic aprotic solvent, with a potassium basic salt, in the presence of water.

2. A process according to claim 1, wherein the preparation of losartan potassium in the crystalline hydrate form comprises:
a) salification reaction to obtain losartan potassium;
b) cooling of the losartan potassium solution to precipitate losartan potassium crystalline hydrate;
c) filtration of the mixture to isolate losartan potassium crystalline hydrate;
d) washing of the losartan potassium crystalline hydrate with a solvent in which losartan potassium crystalline hydrate is insoluble; and
e) drying of the losartan potassium crystalline hydrate.

3. A process according to claim 1, wherein the preparation of losartan potassium in the crystalline form comprises:
a) salification reaction to obtain losartan potassium;
a') azeotropic distillation to remove water;
b) cooling of the losartan potassium solution to precipitate crystalline losartan potassium;
c) filtration of the mixture to isolate crystalline losartan potassium;
d) washing of the crystalline losartan potassium with a solvent in which crystalline losartan potassium is insoluble; and
e) drying of the crystalline losartan potassium.

4. A process according to claims 1, 2 and 3, wherein the organic aprotic solvent is selected from the group comprising acetone, toluene, acetonitrile and ethyl acetate.

5. A process according to claims 1, 2 and 3, wherein the potassium basic salt is selected from potassium hydroxide, potassium carbonate and potassium bicarbonate.

6. A process according to claims 1, 2 and 3, wherein the potassium salt to acid losartan ratio is in a molar ratio ranging from 0.8 to 1.5.

7. A process according to claims 1, 2 and 3, wherein the weight ratio of water to the potassium basic salt ranges from 0.1 to 5.0.

8. A process according to claims 1, 2 and 3, wherein the molar ratio of organic aprotic solvent to acid losartan ranges from 4:1 to 15:1.

9. A process according to claims 1, 2 and 3, wherein the salification reaction is carried out at a temperature ranging from about 15°C to the reflux temperature of the reaction mixture.

10. A process according to claims 2 and 3, wherein the losartan potassium solution is cooled to a temperature lower than 0°C.

11. A process according to claim 3, wherein the water content of the product, after azeotropic distillation, is equal to or lower than 1%.

## Patentansprüche

1. Prozess für die Herstellung von Kalium-Losartan in der Kristallform I oder in der Kristallhydratform mit PXRD Muster mit Peaks bei ungefähr 5,7, 8,9, 13,3, 17,5, 20,0, und 21,1 ± 0,2 Grad 2θ, der die Reaktion einer Dispersion von saurem Losartan in einem organischen aprotischen Lösungsmittel mit einem basischen Kaliumsalz in der Gegenwart von Wasser umfasst.

2. Prozess gemäß Anspruch 1, wobei die Herstellung des Kalium-Losartans in der Kristallhydratform umfasst:
a) Salzbildungsreaktion um Kalium-Losartan zu erhalten;
b) Abkühlen der Kalium-Losartan-Lösung um Kalium-Losartan-Kristallhydrat zu fällen;
c) Filtrieren der Mischung um Kalium-Losartan-Kristallhydrat zu isolieren;
d) Waschen des Kalium-Losartan-Kristallhydrats mit einem Lösungsmittel, in dem Kalium-Losartan-Kristallhydrat unlöslich ist; und
e) Trocknen des Kalium-Losartan-Kristallhydrats.

3. Prozess gemäß Anspruch 1, wobei die Herstellung des Kalium-Losartans in der Kristallform umfasst:
a) Salzbildungsreaktion um Kalium-Losartan zu erhalten;
a') azeotrope Destillation um Wasser zu entfernen;
b) Abkühlen der Kalium-Losartan-Lösung um kristallines Kalium-Losartan zu fällen;
c) Filtrieren der Mischung um kristallines Kalium-Losartan zu isolieren;
d) Waschen des kristallinen Kalium-Losartans mit einem Lösungsmittel, in dem kristallines Kalium-Losartan unlöslich ist; und
e) Trocknen des kristallinen Kalium-Losartans.

4. Prozess gemäß den Ansprüchen 1, 2 und 3, wobei das organische aprotische Lösungsmittel ausgewählt ist aus der Gruppe umfassend Aceton, Toluen, Acetonitril und Ethylacetat.

5. Prozess gemäß den Ansprüchen 1, 2 und 3, wobei das basische Kaliumsalz ausgewählt ist von Kaliumhydroxid, Kaliumcarbonat und Kaliumbicarbonat.

6. Prozess gemäß den Ansprüchen 1, 2 und 3, wobei das Verhältnis von Kaliumsalz zu saurem Losartan in einem molaren Verhältnis im Bereich von 0,8 bis 1,5 ist.

7. Prozess gemäß den Ansprüchen 1, 2 und 3, wobei das Gewichtsverhältnis von Wasser zu dem basischen Kaliumsalz im Bereich von 0,1 bis 5,0 ist.

8. Prozess gemäß den Ansprüchen 1, 2 und 3, wobei das molare Verhältnis von organischem aprotischem Lösungsmittel zu saurem Losartan im Bereich von 4:1 bis 15:1 ist.

9. Prozess gemäß den Ansprüchen 1, 2 und 3, wobei die Salzbildungsreaktion bei einer Temperatur im Bereich von ungefähr 15°C zu der Rückflusstemperatur der Reaktionsmischung durchgeführt wird.

10. Prozess gemäß den Ansprüchen 2 und 3, wobei die Kalium-Losartan-Lösung auf eine Temperatur niedriger als 0°C abgekühlt wird.

11. Prozess gemäß Anspruch 3, wobei der Wassergehalt des Produktes nach azeotroper Destillation gleich oder niedriger als 1% ist.

## Revendications

1. Procédé de préparation de losartan potassique dans une forme cristalline 1 ou dans une forme d'hydrate cristallin ayant un profil PXRD avec des pics à environ 5,7, 8,9, 13,3, 17,5, 20,0 et 21,1 ± 0,2 degrés 20, comprenant la mise en réaction d'une dispersion d'acide de losartan, dans un solvant organique aprotique, avec un sel basique de potassium, en présence d'eau.

2. Procédé selon la revendication 1, dans lequel la préparation de losartan potassique dans une forme d'hydrate cristallin comprend :
a) une réaction de salification pour obtenir du losartan potassique ;
b) un refroidissement de la solution de losartan potassique pour précipiter un hydrate cristallin de losartan potassique ;
c) la filtration du mélange pour isoler l'hydrate cristallin de losartan potassique ;
d) le lavage de l'hydrate cristallin de losartan potassique avec un solvant dans lequel l'hydrate cristallin de losartan potassique est insoluble ; et
e) le séchage de l'hydrate cristallin de losartan potassique.

3. Procédé selon la revendication 1, dans lequel la préparation de losartan potassique dans une forme cristalline comprend :
a) une réaction de salification pour obtenir du losartan potassique ;
a') la distillation azéotropique pour enlever l'eau ;
b) un refroidissement de la solution de losartan potassique pour précipiter un losartan potassique cristallin;
c) la filtration du mélange pour isoler le losartan potassique cristallin ;
d) le lavage du losartan potassique cristallin avec un solvant dans lequel le losartan potassique cristallin est insoluble ; et
e) le séchage du losartan potassique cristallin.

4. Procédé selon les revendications 1, 2 et 3, dans lequel le solvant organique aprotique est sélectionné parmi le groupe comprenant l'acétone, le toluène, l'acétonitrile et l'acétate d'éthyle.

5. Procédé selon les revendications 1, 2 et 3, dans lequel le sel basique de potassium est sélectionné parmi l'hydroxyde de potassium, le carbonate de potassium, et le bicarbonate de potassium.

6. Procédé selon les revendications 1, 2 et 3, dans lequel le rapport du sel de potassium sur l'acide de losartan est un rapport molaire compris entre 0,8 et 1,5.

7. Procédé selon les revendications 1, 2 et 3, dans lequel le rapport en poids de l'eau sur le sel basique de potassium est compris entre 0,1 et 5,0.

8. Procédé selon les revendications 1, 2 et 3, dans lequel le rapport molaire de solvant organique aprotique sur l'acide de losartan est compris entre 4 : 1 et 15 : 1.

9. Procédé selon les revendications 1, 2 et 3, dans lequel la réaction de salification est effectuée à une température comprise entre environ 15°C et la température de reflux du mélange réactionnel.

10. Procédé selon les revendications 2 et 3, dans lequel la solution de losartan potassique est refroidie à une température inférieure à 0°C.

11. Procédé selon la revendication 3, dans lequel le contenu en eau du produit, après distillation azéotropique, est égal à ou inférieur à 1 %.
